(19) Europäisches Patentamt
European Patent Office
Office européen des brevets

(11) **EP 4 746 314 A1**

(12) **EUROPEAN PATENT APPLICATION**
published in accordance with Art. 153(4) EPC

(43) Date of publication:
**20.05.2026 Bulletin 2026/21**

(21) Application number: **23947135.2**

(22) Date of filing: **02.08.2023**

(51) International Patent Classification (IPC):
**H04B 10/071** (2013.01)    **A61B 5/11** (2006.01)

(52) Cooperative Patent Classification (CPC):
**A61B 5/11; H04B 10/071**

(86) International application number:
**PCT/CN2023/110868**

(87) International publication number:
**WO 2025/025187 (06.02.2025 Gazette 2025/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Delta Electronics, Inc.
Taoyuan City 33341 (TW)**

(72) Inventors:
• **CHEN, Chun-yi
Taoyuan City Taiwan 33341 (CN)**

• **WANG, Mu-liang
Taoyuan City Taiwan 33341 (CN)**
• **HO, Ting-wu
Taoyuan City Taiwan 33341 (CN)**
• **TSAI, Feng-jie
Taoyuan City Taiwan 33341 (CN)**
• **LIU, Chia-lung
Taoyuan City Taiwan 33341 (CN)**

(74) Representative: **2K Patent Partnerschaft mbB
Hamburger Allee 26-28
60486 Frankfurt am Main (DE)**

(54) **HUMAN PRESENCE DETECTION APPARATUS, DETECTION SYSTEM AND DETECTION METHOD**

(57)     A personnel presence detection device, a personnel presence detection system, and a personnel presence detection method are disclosed. The personnel presence detection device includes a signal transmitting module for transmitting Wi-Fi signals, a signal receiving module for receiving Wi-Fi reflected signals, a processor for obtaining channel state information (CSI) data based on the Wi-Fi reflected signals, and a computing module for identifying a main radio-frequency path from CSI data set. The computing module processes the CSI data set to determine whether the highest frequency value falls within the respiratory frequency range and generate a first detection result; determine whether the signal variance is greater than a preset value and generate a second detection result; determine whether the number of packets is less than a preset value and generate a third detection result. If any of the three detection results is true, it determines that a person is present.

FIG. 3

# Description

## TECHNICAL FIELD

The present disclosure relates to a detection device, a detection system, and a detection method, and more particularly to a detection device, a detection system, and a detection method for detecting whether a person is present.

## BACKGROUND

The statements in this section merely provide background information related to the present disclosure and do not necessarily constitute prior art.

Under the scorching sun, a vehicle cabin without air conditioning and with doors and windows closed is like an oven. If a child is forgotten to leave in the car, it may cause heat damage or even heat exhaustion, which may be fatal.

Currently, the European New Car Assessment Program (Euro NCAP) has included child presence detection as one of its safety testing standards for new cars, and the US National Highway Traffic Safety Administration (NHTSA) has also included functions of rear seat child reminder system in its New Car Assessment Program (NCAP) proposal.

Currently, many available vehicle occupant detection systems rely solely on respiratory characteristics to detect a person's presence, but fail to detect other characteristics. Furthermore, a person's respiratory characteristics may be less noticeable while inside a car due to factors such as swaying, crying, or adjusting their sitting position. Under these circumstances, traditional vehicle occupant detection systems are prone to misjudgment, rendering them ineffective.

## SUMMARY

The main purpose of the present disclosure is to provide a personnel presence detection device, detection system, and detection method, which can detect the presence of people in a confined space, thereby preventing children from being left alone in the car and preventing thieves from breaking into the car.

In one embodiment, the personnel presence detection device includes:

> a signal transmitting module continuously transmits a plurality of Wi-Fi electromagnetic wave signals outward,
> a signal receiving module continuously receives a plurality of Wi-Fi reflected electromagnetic wave signals,
> a processor controls the signal transmitting module and the signal receiving module, and obtains channel state information data based on the plurality of Wi-Fi reflected electromagnetic wave signals, and

a computing module is connected to the processor, and the computing module identifies a main radio-frequency path from the channel state information data, wherein the main radio-frequency path includes a channel state information data set with the largest number of packets in the channel state information data,

wherein the computing module determines whether the highest frequency value of the channel state information data set falls within a respiratory frequency range through a fast Fourier transform and generates a first detection result, determines whether a signal variance of the channel state information data set is greater than a preset variation amplitude and generates a second detection result, and determines whether the number of packets in the channel state information data set is less than a preset packet threshold and generates a third detection result, and determines that a person is present when one of the first detection result, the second detection result, and the third detection result is true.

As mentioned above, the signal receiving module receives the plurality of Wi-Fi reflected electromagnetic wave signals through a plurality of radio-frequency paths, and the computing module clusters the plurality of Wi-Fi reflected electromagnetic wave signals based on an intensity distribution of each Wi-Fi reflected electromagnetic wave signal in a plurality of subcarriers, and uses the radio-frequency path with the largest number of packets as the main radio-frequency path.

As mentioned above, the computing module clusters the plurality of Wi-Fi reflected electromagnetic wave signals based on a DBSCAN algorithm, and uses the radio-frequency path with the largest number of packets as the main radio-frequency path.

As mentioned above, the respiratory frequency range is from 8 to 22bpm.

As mentioned above, the computing module is implemented in a cloud and connected to the processor through a wired network or a wireless network.

As mentioned above, the personnel presence detection device further a transmission module. The transmission module is connected to the computing module, and the transmission module transmits the detection results with personnel presence outward.

In one embodiment, the personnel presence detection system includes:

> a first Wi-Fi device continuously transmits a plurality of Wi-Fi electromagnetic wave signals,
> a second Wi-Fi device continuously receives a plurality of Wi-Fi reflected electromagnetic wave signals, and obtains channel state information data based on the plurality of Wi-Fi reflected electromagnetic wave signals, and
> a computing module is connected to the second Wi-Fi device, and the computing module receives the channel state information data and identifies a main radio-frequency path from the channel state information data, wherein the main radio-frequency path includes a channel state information data set with the largest number of packets in the channel state information data,

wherein the computing module determines whether the highest frequency value of the channel state information data set falls within a respiratory frequency range through a fast Fourier transform and generate a first detection result, determines whether a signal variance of the channel state information data set is greater than a preset variation amplitude and generates a second detection result, and determines whether the number of packets in the channel state information data set is less than a preset packet threshold and generates a third detection result, and determines that a person is present when one of the first detection result, the second detection result, and the third detection result is true.

As mentioned above, the second Wi-Fi device receives the plurality of Wi-Fi reflected electromagnetic wave signals through a plurality of radio-frequency paths, and the computing module clusters the plurality of Wi-Fi reflected electromagnetic wave signals based on an intensity distribution of each Wi-Fi reflected electromagnetic wave signal in a plurality of subcarriers, and uses the radio-frequency path with the largest number of packets as the main radio-frequency path.

As mentioned above, the computing module clusters the plurality of Wi-Fi reflected electromagnetic wave signals based on a DBSCAN algorithm, and uses the radio-frequency path with the largest number of packets as the main radio-frequency path.

As mentioned above, the personnel presence detection system further includes a remote device. the computing module is implemented in a cloud and connected to the second Wi-Fi device through a wired network or a wireless network, and the remote device connects to the computing module through the wired network and the wireless network to query the detection results with personnel presence.

In one embodiment, the personnel presence detection method includes steps of:

(a) continuously transmitting a plurality of Wi-Fi electromagnetic wave signals outward,
(b) continuously receiving, by a Wi-Fi device, a plurality of Wi-Fi reflected electromagnetic wave signals,
(c) obtaining, by the Wi-Fi device, channel state information data based on the plurality of Wi-Fi reflected electromagnetic wave signals,
(d) identifying a main radio-frequency path from the channel state information data, wherein the main radio-frequency path comprises a channel state information data set with the largest number of packets in the channel state information data,

(e) determining whether the highest frequency value of the channel state information data set falls within a respiratory frequency range through a fast Fourier transform and generate a first detection result,
(f) determining whether a signal variance of the channel state information data set is greater than a preset variation amplitude and generate a second detection result,
(g) determining whether the number of packets in the channel state information data set is less than a preset packet threshold and generate a third detection result, and
(h) determining that a person is present when one of the first detection result, the second detection result, and the third detection result is true.

As mentioned above, step (a) is that the Wi-Fi device is configured to continuously transmit the plurality of Wi-Fi electromagnetic wave signals outward, and step (b) is that the same Wi-Fi device is configured to continuously receive the plurality of Wi-Fi reflected electromagnetic wave signals.

As mentioned above, step (b) comprises a step of: receiving the plurality of Wi-Fi reflected electromagnetic wave signals through a plurality of radio-frequency paths, and step (d) comprises a step of: clustering the plurality of Wi-Fi reflected electromagnetic wave signals based on an intensity distribution of each Wi-Fi reflected electromagnetic wave signal in a plurality of subcarriers, and using the radio-frequency path with the largest number of packets as the main radio-frequency path.

As mentioned above, step (d) comprises a step of: clustering the plurality of Wi-Fi reflected electromagnetic wave signals based on a DBSCAN algorithm, and using the radio-frequency path with the largest number of packets as the main radio-frequency path.

As mentioned above, the personnel presence detection method further includes a step of:

(i) connecting the computing module to a remote device through a wired network or a wireless network, and querying the detection results with personnel presence.

Compared with related technologies, the detection device, detection system, and detection method of the present disclosure simultaneously detect a person's respiratory characteristics, small movement characteristics, and large movement characteristics based on Wi-Fi signals, thereby more accurately determining whether there is a person in the confined space.

BRIEF DESCRIPTION OF DRAWINGS

FIG. 1 is a specific diagram of a detection operation according to the present disclosure.

FIG. 2 is a specific diagram of a detection operation according to the present disclosure.

FIG. 3 is a block diagram of a personnel presence detection device according to the present disclosure.

FIG. 4 is a flowchart of a personnel presence detection method according to the present disclosure.

FIG. 5 is a schematic diagram of a multipath Wi-Fi signal according to the present disclosure.

FIG. 6 is a schematic diagram of channel state information (CSI) data according to the present disclosure.

FIG. 7A to FIG. 7D are schematic diagrams of respiratory characteristic conversion according to the present disclosure.

FIG. 8 is a schematic diagram of small movement characteristics of the present disclosure.

FIG. 9 is a schematic diagram of large movement characteristics of the present disclosure.

FIG. 10 is a schematic diagram of a person presence detection system according to the present disclosure.

Description of Reference Numerals in Drawings

1: personnel presence detection device

11: signal transmitting module

12: signal receiving module

13: processor

14: computing module

15: transmission module

20: confined space

2: person

3: vehicle

4: house

5: first Wi-Fi device

6: second Wi-Fi device

7: computing module

8: remote device

P1-P6: packages

RF1: first RF path

RF2: second RF path

RF3: third RF path

T1: first time interval

T2: second time interval

S40-S50: detection steps

DETAILED DESCRIPTION

Reference will now be made to the drawing figures to describe the present disclosure in detail. It will be understood that the drawing figures and exemplified embodiments of present disclosure are not limited to the details thereof.

In order to accurately detect whether a person has been left in a vehicle, the present disclosure provides a person presence detection device (hereinafter abbreviated as "detection device". The detection device processes and detects signal packets in a confined space and determines the presence of a person when human-related characteristics are detected in these signal packets. The detection device of the present disclosure is used to process and detect signal packets in a confined space, and determines the presence of a person when characteristics related to a person are detected in these signal packets.

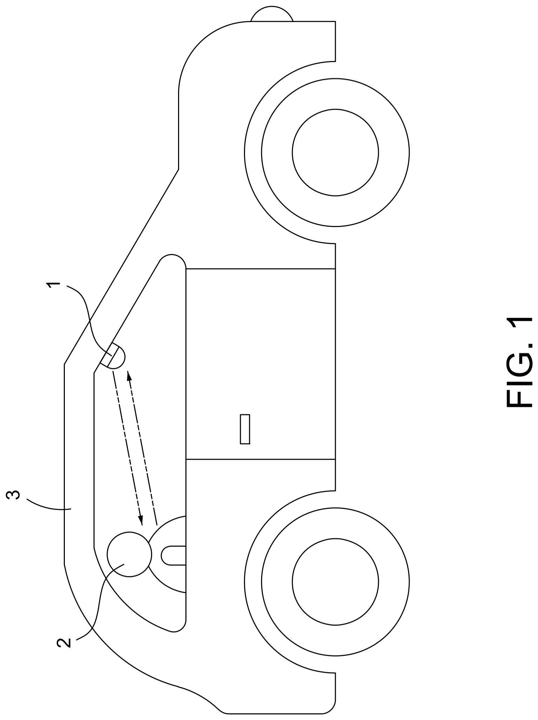

Please refer to FIG. 1, which shows a specific diagram of a detection operation according to the present disclosure. In the embodiment of FIG. 1, a confined space is, for example, a cabin of a vehicle 3, and the detection device 1 is disposed in the cabin to detect a person 2 in the cabin.

In one embodiment, the detection device 1 can be manually activated by a user, or connected to an in-vehicle system of the vehicle 3. When the vehicle 3 is turned off, the driver exits, and the doors are locked, the detection device 1 is activated manually by the user or automatically by the in-vehicle system. Therefore, the detection device 1 can detect the presence of children in the vehicle 3, thereby preventing careless drivers from leaving children unattended. Since the detection device 1 of the present disclosure detects the presence of person 2 while the vehicle 3 is turned off, no misjudgment will occur due to vibrating vehicle components.

In particular, according to FBI statistics, over 3,000 burglaries occur daily in the United States, and it's conceivable that similar situations exist in other countries. The detection device 1 of the present disclosure is primarily used to detect the presence of a person 2 within the interior of a vehicle 3. Furthermore, since a house is also a confined space, the detection device 1 of the present disclosure may also be installed within the house to detect thieves, thereby increasing home security.

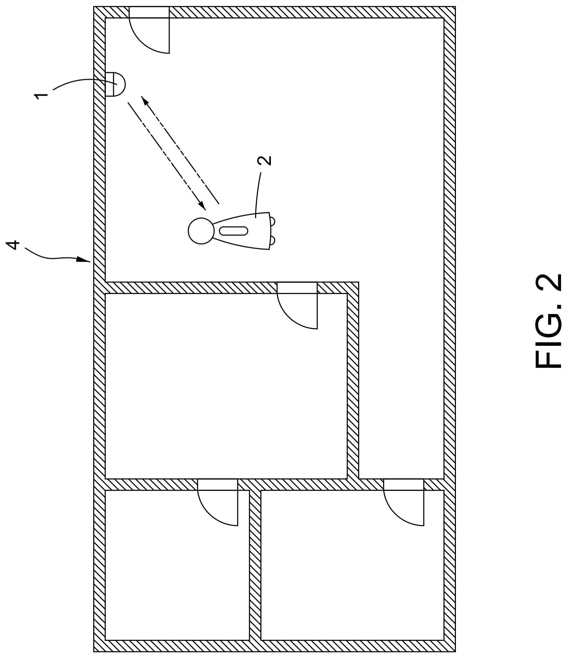

Please refer to FIG. 2, which shows a specific diagram of a detection operation according to the present disclosure. In the embodiment of FIG. 2, the confined space is, for example, a house 4, and the detection device 1 is disposed in one or more specific rooms in the house 4 to detect whether a thief is present in the house 4.

In one embodiment, the user can activate the detection device 1 when they are away and are certain no one is home. In another embodiment, the detection device 1 may be connected to a home security system. In this embodiment, after being manually activated by the user or automatically activated by the security system, the detection device 1 can continuously detect signal packets within the house 4 and alert the user if a signal packet shows characteristics associated with a person, thereby detecting a thief.

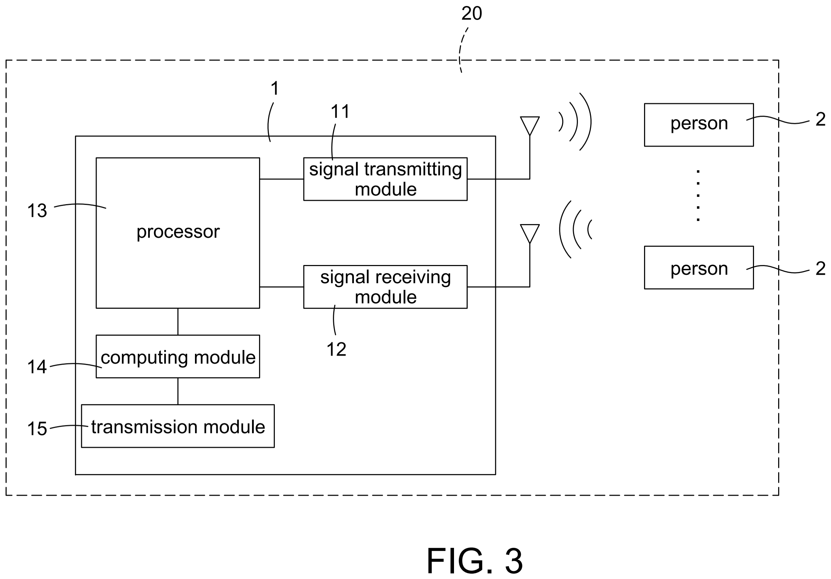

Please refer to FIG. 3, which shows a block diagram of a personnel presence detection device according to the present disclosure. As shown in FIG. 3, the detection device 1 of the present disclosure at least includes a signal transmitting module 11, a signal receiving module 12, a processor 13, and a computing module 14. The processor 13 is electrically connected to the signal transmitting module 11 and the signal receiving module 12. The processor 13 controls the signal transmitting module 11 to transmit signal packets outward and controls the signal receiving module 12 to receive reflected signal packets.

In one embodiment, the computing module 14 is disposed within detection device 1 and is electrically connected to the processor 13. In another embodiment, the computing module 14 is disposed externally to the detection device 1 (e.g., on an external hard drive, external computer, or cloud) and is connected to the processor 13 of the detection device 1 through a wired or wireless connection. However, the above are only some specific implementation examples of the present disclosure, and are not limited thereto.

In one embodiment, the detection device 1 may be a Wi-Fi device. The signal transmitting module 11 and the signal receiving module 12 may be respectively a transmitting antenna and a receiving antenna of the Wi-Fi device. The detection device 1 continuously transmits a plurality of Wi-Fi electromagnetic wave signals outward through the signal transmitting module 11. After the plurality of Wi-Fi electromagnetic wave signals are transmitted to the detection device 1, the plurality of Wi-Fi electromagnetic wave signals will radiate in the confined space 20 and be reflected after hitting a wall, object or a person 2 in the confined space 20. The detection device 1 continuously receives a plurality of Wi-Fi reflected electromagnetic wave signals from the confined space 20 through the signal receiving module 12. In the present disclosure, the detection device 1 processes the plurality of Wi-Fi reflected electromagnetic wave signals to determine whether a person 2 is present in the confined space 20.

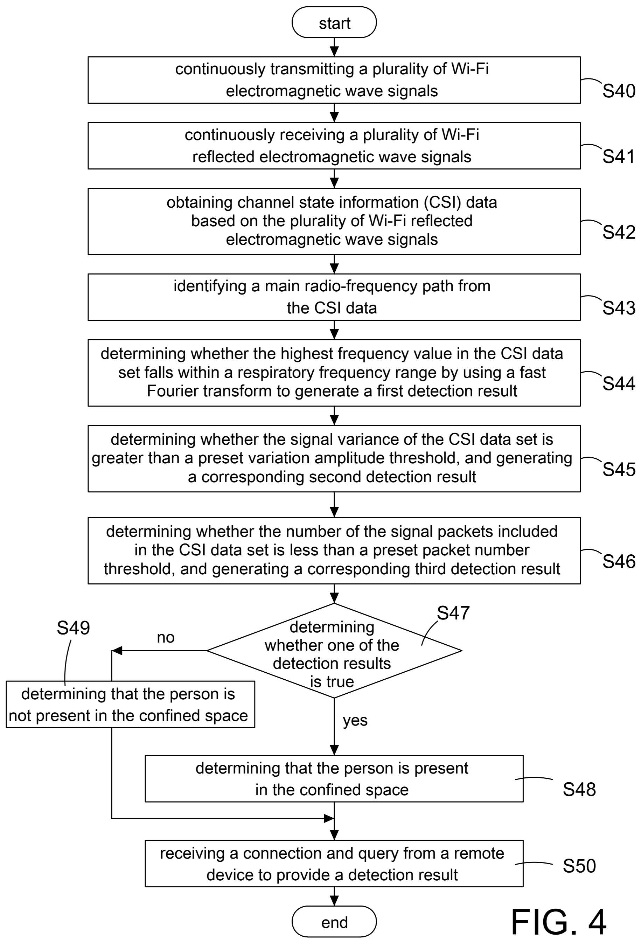

Please refer to FIG. 4, which shows a flowchart of a personnel presence detection method according to the present disclosure, and also refer to FIG. 3. FIG. 4 discloses the specific steps of the method for detecting presence of a person (hereinafter referred to as the detection method) of the present disclosure, which can be applied to the detection device 1 shown in FIG. 3.

Specifically, the processor 13 of the detection device 1 may be, but is not limited to, a central processing unit (CPU), a microcontroller unit (MCU), a programmable logic controller (PLC), a system on a chip (SoC), or a field programmable gate array (FPGA). The processor 13 stores computer-executable program code. When the detection device 1 is powered on to be activated and the processor 13 executes the computer-executable program code, the processor 13 controls the signal transmitting module 11, the signal receiving module 12, and the computing module 14 to jointly execute steps shown in FIG. 4 to implement the detection method of the present disclosure.

As shown in FIG. 4, after the detection device 1 is activated (i.e., the user determines that no one is in the confined space 20), the detection device 1 controls the signal transmitting module 11 to continuously transmit the plurality of Wi-Fi electromagnetic wave signals outward (step S40), and controls the signal receiving module 12 to continuously receive the plurality of Wi-Fi reflected electromagnetic wave signals from the outside (step S41).

In another embodiment, step S40 and step S41 are performed by the signal transmitting module 11 and the signal receiving module 12 of the same detection device (e.g., the detection device 1 shown in FIG. 3) respectively, to transmit the plurality of Wi-Fi electromagnetic wave signals and receive the plurality of Wi-Fi reflected electromagnetic wave signals. Therefore, the detection method of the present disclosure can be implemented in the confined space 20 using a single detection device 1. By using a single detection device 1 to transmit and receive signals by itself, the implementation cost of the detection method can be reduced, as well as the space required to install the detection device 1.

In another embodiment, step S40 involves the signal transmitting module of the first detection device transmitting the plurality of Wi-Fi electromagnetic wave signals, while step S41 involves the signal receiving module of the second detection device receiving the plurality of Wi-Fi reflected electromagnetic wave signals. Therefore, the detection method of the present disclosure can be implemented in confined space 20 using a plurality of detection devices (e.g., a Wi-Fi Mesh composed of a plurality of Wi-Fi devices). By using a plurality of detection devices to transmit and receive signals by itself, the detection range can be expanded and the detection accuracy can be increased.

In the present disclosure, the signal transmitting module 11 continuously transmits the plurality of Wi-Fi electromagnetic wave signals at a designated frequency, and the signal receiving module 12 continuously receives the plurality of Wi-Fi reflected electromagnetic wave signals at a designated frequency. Therefore, the plurality of Wi-Fi electromagnetic wave signals and the plurality of Wi-Fi reflected electromagnetic wave signals are time-sequential, rather than being transmitted or received simultaneously. Since these signal packets are time-sequential, after step S41, the processor 13 of the detection device 1 can obtain channel state information (CSI) data based on the plurality of Wi-Fi reflected electromagnetic wave signals (step S42).

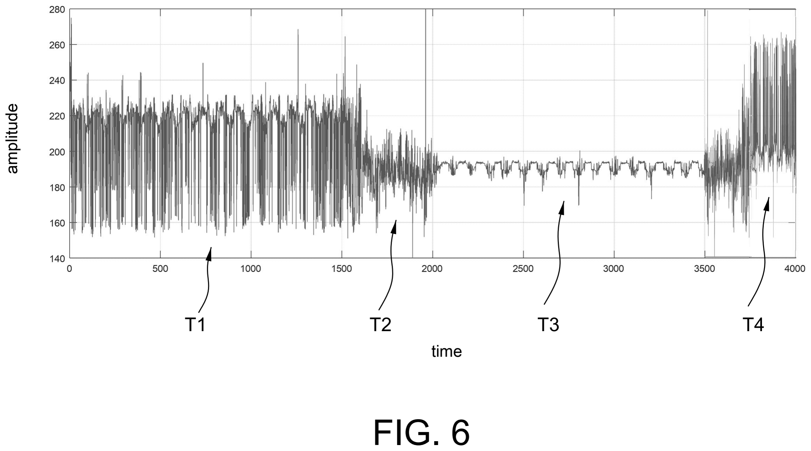

In the field of wireless communications, CSI data (as shown in FIG. 6) indicates the known channel characteristics of a communication link and describes how a signal packet is transmitted from the transmitter to the receiver through a channel (or path). Since CSI data can be used to determine the energy attenuation of a signal packet over distance, in the present disclosure, the detection device 1 first converts the plurality of Wi-Fi reflected electromagnetic wave signals into CSI data for observation. After obtaining the CSI data, the processor 13 provides the CSI data to the computing module 14, which identifies the main radio-frequency (RF) path from the CSI data (step S43). In the present disclosure, the main RF path refers to the path used to transmit a CSI data set with the largest number of packets.

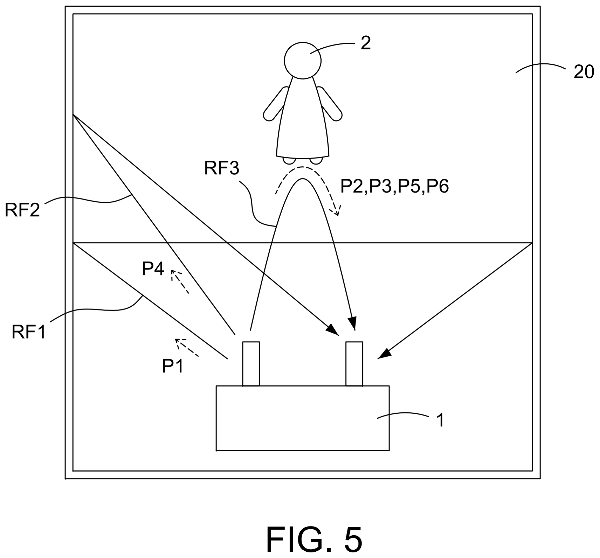

Please refer to FIG. 5, which shows a schematic diagram of a multipath Wi-Fi signal according to the present disclosure. In the present disclosure, the signal transmitting module 11 transmits the plurality of Wi-Fi electromagnetic wave signals by using a scattering transmission manner. These signal packets are transmitted and reflected along the same or different paths within the confined space 20. Therefore, the signal receiving module 12 can receive the plurality of Wi-Fi reflected electromagnetic wave signals through the plurality of RF paths, in particular, the RF path through which the most Wi-Fi reflected electromagnetic wave signals pass is the main RF path.

Specifically, after being scattered, these signal packets are reflected within the confined space 20 upon striking walls, objects, or the person 2. Since the signal transmitting module 11 continuously transmits signal packets outward, the signal receiving module 12 can continuously receive reflected packets through the same or different RF paths. The present disclosure uses a clustering method to identify a main RF path containing the largest number of signal packets among the plurality of RF paths. Based on the plurality of signal packets (CSI data) contained within the main RF path, detection is performed to determine whether they contain characteristics unique to person 2, such as respiratory characteristics, small movement characteristics, or large movement characteristics.

In the embodiment of FIG. 5, a first signal packet P1 is transmitted along a first RF path RF1 within the confined space 20 and received by the signal receiving module 12. A fourth signal packet P4 is transmitted along a second RF path RF2 within the confined space 20 and received by the signal receiving module 12. A second signal packet P2, a third signal packet P3, a fifth signal packet P5, and a sixth signal packet P6 are transmitted along a third RF path RF3 within the confined space 20 and received by the signal receiving module 12. In other words, the second signal packet P2, the third signal packet P3, the fifth signal packet P5, and the sixth signal packet P6 travel the same transmission path within the confined space 20, but differ from the transmission paths of the first signal packet P1 and the fourth signal packet P4.

By utilizing multipath scattering transmission, the detection device 1 of the present disclosure ensures that every corner of the confined space 20 is detected, thereby reducing the probability of misjudgments. Furthermore, since the plurality of signal packets are not transmitted through a single path, even when used within the confined space 20, there is no risk of packet loss or interference affecting detection results.

Please refer to FIG. 6, which shows a schematic diagram of channel state information (CSI) data according to the present disclosure. As shown in FIG. 6, in certain situations (e.g., during a first time interval T1 when the person 2 has just seated), significant multipath transmission effects may occur, resulting in large fluctuations, which makes it difficult to distinguish the characteristics of the person 2. If the original CSI data is directly used for detection (e.g., detecting the respiratory characteristic of the person 2), misjudgments may occur.

In order to overcome above-mentioned problems, the detection method of the present disclosure first clusters the plurality of received Wi-Fi electromagnetic wave signals to obtain the main RF path containing the largest number of signal packets, and then processes the plurality of signal packets within the main RF path. Therefore, this method filters out unnecessary noise, thereby making it easier to detect the characteristics of the person 2.

In particular, in certain situations (e.g., during a third time interval T3 when the person 2 is quietly seated), the influence of multipath transmission is minimal, resulting in small and stable fluctuations, which makes the presence of the person 2 more distinguishable. In this case, the computing module 14 can directly use the original CSI data for detection (e.g., detecting the respiratory characteristic of the person 2) without performing clustering.

As mentioned above, the plurality of Wi-Fi reflected electromagnetic wave signals may travel the same or different RF paths within the confined space 20. In order to effectively obtain useful characteristics from Wi-Fi reflected electromagnetic wave signals, the detection method of the present disclosure first identifies the main RF path containing the largest number of signal packets and then performs detection based on the CSI data set within the main RF path (i.e., the set of all Wi-Fi reflected electromagnetic wave signals transmitted along the main RF path).

In the present disclosure, the computing module 14 clusters the plurality of Wi-Fi reflected electromagnetic wave signals based on their frequency variations. The signal packets with the same or similar frequency variations are clustered together. Since the signal packets clustered together have the same or similar frequency variations, they share the same transmission path.

In one embodiment, the computing module 14 converts each Wi-Fi reflected electromagnetic wave signal into a plurality of subcarriers based on the CSI data to observe the intensity distribution of these Wi-Fi reflected electromagnetic wave signals, and clusters the plurality of Wi-Fi reflected electromagnetic wave signals based on the intensity distribution. Specifically, the computing module 14 clusters the plurality of signal packets with the same or similar intensity distribution of the Wi-Fi reflected electromagnetic wave signals in the plurality of subcarriers into the same cluster (i.e., belonging to the same RF path), and regards the RF path with the largest number of packets as the main RF path.

In another embodiment, the computing module 14 directly clusters the plurality of Wi-Fi reflected electromagnetic wave signals based on a DBSCAN algorithm, and uses the RF path with the largest number of packets as the main RF path. However, the above are only some specific implementation examples of the present disclosure, and are not limited thereto.

Returning to FIG. 3 and FIG. 4, after step S43, the computing module 14 obtains the CSI data set for the main RF path (i.e., CSI data for all signal packets transmitted through the main RF path) using the clustering manner. Afterward, the CSI data set is processed using a fast Fourier transform (FFT) to determine whether the highest frequency value in the CSI data set falls within the respiratory frequency range to generate a first detection result (step S44).

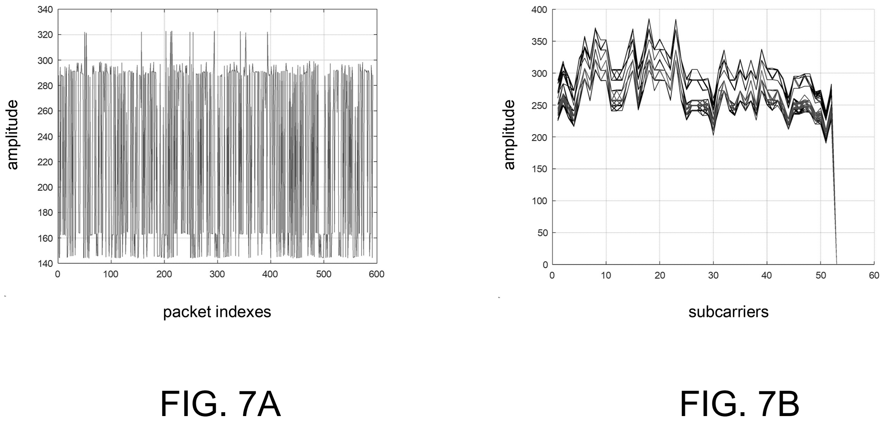

Please refer to FIG. 7A to FIG. 7D, which show schematic diagrams of respiratory characteristic conversion according to the present disclosure. As shown in FIG. 7A, in certain situations, the CSI data can be subject to significant multipath transmission and large fluctuations, thereby making it difficult to clearly identify the respiratory characteristics of the person 2. Therefore, as shown in FIG. 7B, the computing module 14 converts the plurality of Wi-Fi reflected electromagnetic wave signals into a plurality of subcarriers based on the CSI data to observe the signal intensity distribution, and these Wi-Fi reflected electromagnetic wave signals are clustered based on the intensity distribution. That is, the plurality of Wi-Fi reflected electromagnetic wave signals with the same or similar intensity distribution are clustered together (i.e., belonging to the same RF path).

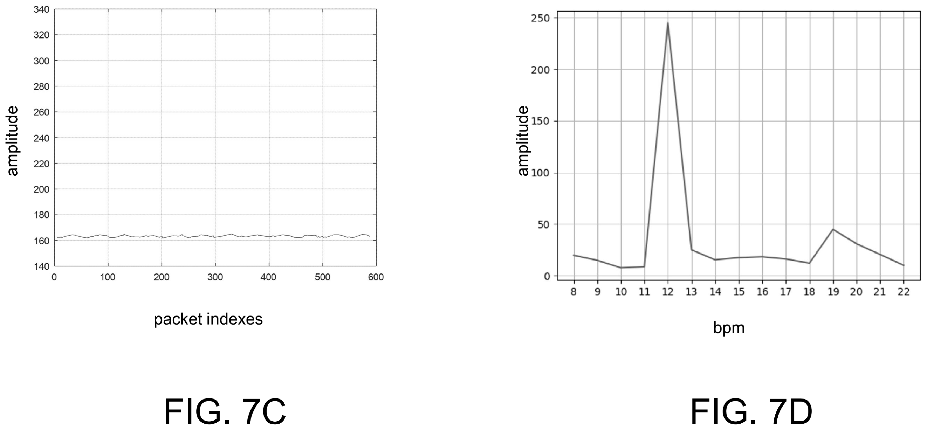

Afterward, as shown in FIG. 7C, the computing module 14 retains only the plurality of Wi-Fi reflected electromagnetic wave signals (i.e., the CSI data set) included in the main RF path. Finally, as shown in FIG. 7D, the computing module 14 converts the CSI data set into frequency domain data using a fast Fourier transform (FFT) and determines whether the highest frequency value in the CSI data set falls within a preset respiratory frequency range.

In one embodiment, the respiratory frequency range is a user-defined range corresponding to the respiratory frequency of the person 2, for example, 8 bpm to 22 bpm, but this is not limited thereto. In the embodiment of FIG. 7D, the highest frequency value in the CSI data set is 12 bpm, falling within the preset respiratory frequency range of 8 bpm to 22 bpm. Therefore, the computing module 14 can determine that the person 2 is present in the confined space 20.

In one embodiment, the computing module 14 sets the first detection result as "true" when determining that the highest frequency value of the CSI data set falls within the respiratory frequency range (i.e., determining that the person 2 is present in the confined space 20), and sets the first detection result as "false" when determining that the highest frequency value of the CSI data set does not fall within the respiratory frequency range (i.e., determining that the person 2 is not present in the confined space 20).

Returning to FIG. 4, after step S44, the computing module 14 further determines whether the signal variance of the CSI data set is greater than a preset variation amplitude, and generates a corresponding second detection result (step S45).

As shown in FIG. 6, in certain situations (e.g., during a second time interval T2 in FIG. 6), if the person 2 makes small movements within the confined space 20, such as waving or crying, the detection device 1 may be unable to accurately detect respiratory characteristics based on the CSI data. In order to avoid misjudgments caused by small movements of the person 2, the detection device 1 of the present disclosure further detects small movement characteristics of the person 2 based on the CSI data.

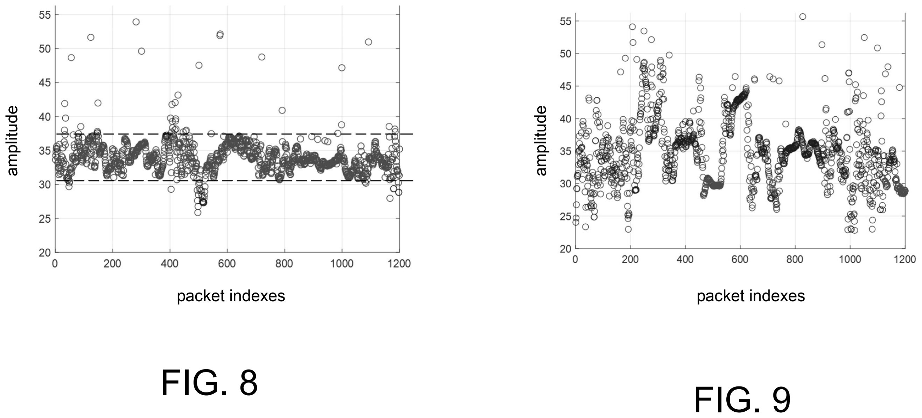

Please refer to FIG. 8, which shows a schematic diagram of small movement characteristics of the present disclosure. In FIG. 8, the dark circles represent the amplitude of signal packets transmitted through the main RF path, while the light circles represent the amplitude of signal packets transmitted through other RF paths.

The small movement characteristic refers to the signal variance of the CSI data set. In the present disclosure, the user can set a desired variation amplitude based on variables such as the size and shape of the confined space 20, and the number, type, and size of inanimate objects placed within. Therefore, the computing module 14 can determine whether a person 2 is present in the confined space 20 based on whether the signal variance of the CSI data set exceeds this variation amplitude.

In particular, the user can reset the sensitivity of detection device 1 based on environmental variables. If the user believes that the detection result produced by the detection device 1 at its current sensitivity is not as expected (for example, mistaking an inanimate object for a person), the user can adjust the sensitivity of the detection device 1 (e.g., modify the variation amplitude) based on the environmental conditions of the confined space 20 (e.g., whether equipment or machinery has been added or removed), which allows the detection device 1 to be calibrated to increase detection accuracy.

Specifically, the variation amplitude refers to the degree of change in signal amplitude. The computing module 14 calculates an amplitude difference between the highest and lowest peaks of the plurality of signal packets in the CSI data set and compares the amplitude difference with a preset variation amplitude. If this amplitude difference is greater than the preset variation amplitude, it indicates that the person 2 is present in the confined space 20 and is performing a small movement.

In one embodiment, the computing module 14 may calculate the signal variance according to the following formula:

$$ \mathrm{Var(Q)} = \sum_{i=1}^{|Q|} \frac{(\bar{q}_i - \bar{q})^2}{|Q|} $$

In the above formula, Var is the signal variance, Q is the CSI data set, wherein $Q = \{q_i = [q_i^1, q_i^2, \ldots, q_i^m]\}$ , and m is the number of the subcarriers. $\bar{q}_i = \sum_{j=1}^m \frac{q_i^j}{m}$ and $\bar{q} = \sum_{i=1}^{|Q|} \frac{\bar{q}_i}{|Q|}$

In one embodiment, the computing module 14 outputs the second detection result as "true" when the signal variance of the CSI data set is greater than the preset variation amplitude (i.e., it is determined that the person 2 is present in the confined space 20), and outputs the second detection result as "false" when the signal variance of the CSI data set is less than or equal to the preset variation amplitude (i.e., it is determined that the person 2 is not present in the confined space 20).

Returning to FIG. 4, after step S45, the computing module 14 further determines whether the number of the signal packets included in the CSI data set is less than a preset number threshold, and generates a corresponding third detection result (step S46).

As shown in FIG. 6, in some situations (e.g., during the fourth time interval T4 in FIG. 6), the person 2 may make large movements within the confined space 20, such as getting up and getting off a vehicle. In such situations, the detection device 1 may be unable to detect respiratory characteristics based on the CSI data. In order to avoid misidentification due to large movements by the person 2, the detection device 1 of the present disclosure further detects large movement characteristics of the person 2 based on the CSI data.

Please also refer to FIG. 9, which shows a schematic diagram of large movement characteristics of the present disclosure. In FIG. 9, the dark circles represent the amplitude of signal packets transmitted through the main RF path, while the light circles represent the amplitude of signal packets transmitted through other RF paths. As seen from FIG. 9, when the person 2 makes large movements, the number of the signal packets transmitted through the main RF path is relatively small compared to the embodiment of FIG. 8.

Since the number of the signal packets transmitted along the same path decreases significantly when the person 2 makes large movements, even the main RF path will still contain fewer packets than expected. In the present disclosure, the user can preset a desired number threshold based on variables such as the size and shape of the confined space 20, and the number, type, and size of inanimate objects placed within. Therefore, the computing module 14 can determine whether a person 2 is present in the confined space 20 based on whether the number of the signal packets included in the CSI data set is less than the preset number threshold.

As mentioned above, when the user believes that the detection result produced by the detection device 1 is not as expected, the user can adjust the sensitivity of the detection device 1 according to the environmental conditions of the confined space 20 (for example, modify the number threshold), which allows the detection device 1 to be calibrated to increase detection accuracy.

Specifically, if the computing module 14 identifies a main RF path with a relatively large number of packets in step S43 of FIG. 4, but the number of the signal packets included in this main RF path is less than the preset number threshold, it means that a person 2 is present in the confined space 20 and the person 2 is making a large movement.

In one embodiment, the computing module 14 outputs the third detection result as "true" when the number of the signal packets included in the CSI data set is less than the number threshold (i.e., it is determined that the person 2 is present in the confined space 20), and outputs the third detection result as "false" when the number of the signal packets included in the CSI data set is greater than or equal to the number threshold (i.e., it is determined that the person 2 is not present in the confined space 20).

In particular, the present disclosure simultaneously detects respiratory characteristics, small movement characteristics, and large movement characteristics based on the CSI data in the main RF path, thereby avoiding misidentification. However, the detection of these three characteristics does not follow a specific order, that is, steps S44, S45, S46 in FIG. 4 are not executed in a fixed order and are not limited to the one shown in FIG. 4.

Returning to FIG. 4, after step S46, the computing module 14 determines whether one of the first detection result (corresponding to the respiratory characteristics), the second detection result (corresponding to the small movement characteristics), and the third detection result (corresponding to the large movement characteristics) is true (step S47). In the present disclosure, the computing module 14 simultaneously detects the respiratory characteristics, the small movement characteristics, and the large movement characteristics based on the same CSI data. If any of the detection results is true, it determines that the person 2 is present in the confined space 20 (step S48). Conversely, if the first detection result, the second detection result, and the third detection result are all false, it means that the computing module 14 cannot detect the respiratory characteristics, the small movement characteristics, or the large movement characteristics from the CSI data. In this case, the computing module 14 determines that the person 2 is not present in the confined space 20 (step S49).

After generating the detection results of whether the person 2 is present or absent, the computing module 14 of the present disclosure can receive a connection and query from a remote device through a wired or wireless method to provide a detection result of whether the person 2 is present (step S50).

Specifically, as shown in FIG. 3, the detection device 1 may further include a transmission module 15 connected to the computing module 14. The transmission module 15 may be, for example, a Wi-Fi module, a Bluetooth module, an infrared module, or a connector module, for connecting to an external remote device through wired or wireless manners. In the present disclosure, a user can use a remote device to connect to the detection device 1 (or a cloud with the computing module 14 is provided) to remotely receive an alert notification indicating the presence of the person 2. Therefore, this allows the user to receive immediate alert notifications, whether a child is left alone in a vehicle 3 or a thief breaks into a house 4 with no one inside, thereby preventing unforeseen circumstances.

In one embodiment, the detection device 1 of the present disclosure may also be provided with a display module (not shown). After the computing module 14 generates a detection result indicating the presence or absence of the person 2, the detection result may be directly displayed through the display module (e.g., a display screen or a speaker).

In the above-mentioned embodiment, the detection device 1 is a single Wi-Fi device. The detection method of the present disclosure is implemented by using the signal transmitting module 11 and the signal receiving module 12 within the detection device 1 to transmit and receive signal packets by itself. Therefore, the detection method of the present disclosure can be implemented without requiring the signal transmitting module 11 and the signal receiving module 12 within the detection device 1 to share a common oscillator.

In the above-mentioned embodiment, the signal transmitting module 11 and the signal receiving module 12 may use the 5.8 GHz frequency band to connect to the user's mobile device (not shown) to provide networking function, and simultaneously use the 5.8 GHz frequency band to transmit and receive Wi-Fi electromagnetic wave signals, thereby detecting whether a person is present in the confined space 20.

In addition to the embodiment of realizing personnel detection by using a single detection device 1 to transmit and receive signal packets, the present disclosure further discloses a personnel presence detection system (hereinafter referred to as the detection system), which uses at least two detection devices to transmit and receive signal packets to realize the detection method of the present disclosure.

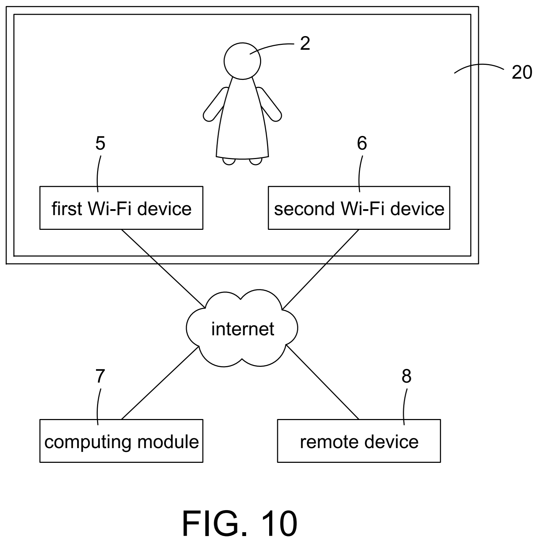

Please refer to FIG. 10, which shows a schematic diagram of a person presence detection system according to the present disclosure. In the embodiment of FIG. 10, the detection system includes a first Wi-Fi device 5, a second Wi-Fi device 6, and a computing module 7, wherein the first Wi-Fi device 5 and the second Wi-Fi device 6 are disposed in the same confined space 20. In this embodiment, the first Wi-Fi device 5, the second Wi-Fi device 6, and the computing module 7 respectively execute corresponding computer-executable codes to collectively implement steps of the detection method shown in FIG. 4.

In one embodiment, the computing module 7 is connected to the first Wi-Fi device 5 and the second Wi-Fi device 6 through a wired or wireless connection. In another embodiment, the computing module 7 is connected to a Wi-Fi device (hereinafter, using the second Wi-Fi device 6 as an example) for receiving Wi-Fi reflected electromagnetic wave signals through a wired or wireless connection.

In this embodiment, the first Wi-Fi device 5 and the second Wi-Fi device 6 are connected to each other through the 2.4 GHz frequency band, thereby establishing a Wi-Fi mesh network within the confined space 20. Furthermore, the first Wi-Fi device 5 and the second Wi-Fi device 6 are connected to a user's mobile device (not shown) through the 5.8 GHz frequency band to provide networking function. Similar to the previous embodiment, the first Wi-Fi device 5 and the second Wi-Fi device 6 also use the 5.8 GHz frequency band to transmit and receive Wi-Fi electromagnetic wave signals, thereby detecting whether a person 2 is present in the confined space 20.

In the embodiment of FIG. 10, after the user leaves the confined space 20, the detection system can be activated. At this time, the detection system controls the first Wi-Fi device 5 to continuously transmit a plurality of Wi-Fi electromagnetic wave signals and controls the second Wi-Fi device 6 to continuously receive a plurality of Wi-Fi reflected electromagnetic wave signals. Furthermore, the detection system can use the second Wi-Fi device 6 to obtain the CSI data based on the plurality of Wi-Fi reflected electromagnetic wave signals. The Wi-Fi electromagnetic wave signals, the Wi-Fi reflected electromagnetic wave signals, and the CSI data are the same as those described in the previous embodiment and will not be further described here.

In the first embodiment, the computing module 7 may be installed in the second Wi-Fi device 6 responsible for receiving the Wi-Fi reflected electromagnetic wave signal, thereby being implemented by the second Wi-Fi device 6. In the second embodiment, the computing module 7 may be independently installed in the confined space 20 (e.g., implemented by a smart mobile device, a tablet computer, or a personal computer) and connected to the first Wi-Fi device 5 and/or the second Wi-Fi device 6 through wired or wireless manners.

In the third embodiment, the computing module 7 may be implemented in the cloud (e.g., implemented by a cloud server) and connected to the first Wi-Fi device 5 and/or the second Wi-Fi device 6 through a wired or wireless network. Therefore, the computing module 7 can receive the CSI data from the first Wi-Fi device 5 or the second Wi-Fi device 6 and identify the main RF path from the CSI data. The main RF path is the same as in the previous embodiment and will not be further described here.

As in the previous embodiment, after the computing module 7 obtains the CSI data and identifies the main RF path, it processes the CSI data set and performs the following operations:

(1) detecting respiratory characteristics and generating the first detection result, (2) detecting small movement characteristics and generating the second detection result, and (3) detecting large movement characteristics and generating the third detection result. The respiratory characteristics, small movement characteristics, large movement characteristics, and their detection methods are the same as those described in the previous embodiment and are not further described here.

When the computing module 7 determines that at least one of the first detection result, the second detection result, and the third detection result is true, it generates a detection result indicating that a person is present in the confined space. Conversely, when the first detection result, the second detection result, and the third detection result are all false, the computing module 7 generates a detection result indicating that no person is present.

In the present disclosure, the user can remotely operate the remote device 8 (e.g., a smart mobile device) and connect to the computing module 7 through a wired or wireless network to query the computing module 7 for the detection results.

In one embodiment, a user can log into the computing module 7 through the remote device 8. When the computing module 7 determines that a person is present in confined space 20 based on the first detection result, the second detection result, and the third detection result, it can immediately and automatically send an alert message to the remote device 8 to notify the user, thereby preventing potential problems.

Although the present disclosure has been described with reference to the preferred embodiment thereof, it will be understood that the present disclosure is not limited to the details thereof. Various substitutions and modifications have been suggested in the foregoing description, and others will occur to those of ordinary skill in the art. Therefore, all such substitutions and modifications are intended to be embraced within the scope of the present disclosure as defined in the appended claims.

## Claims

1. A personnel presence detection device, comprising:

a signal transmitting module configured to continuously transmit a plurality of Wi-Fi electromagnetic wave signals outward,
a signal receiving module configured to continuously receive a plurality of Wi-Fi reflected electromagnetic wave signals,
a processor configured to control the signal transmitting module and the signal receiving module, and obtain channel state information data based on the plurality of Wi-Fi reflected electromagnetic wave signals, and
a computing module connected to the processor, and the computing module configured to identify a main radio-frequency path from the channel state information data, wherein the main radio-frequency path comprises a channel state information data set with the largest number of packets in the channel state information data,
wherein the computing module is configured to determine whether the highest frequency value of the channel state information data set falls within a respiratory frequency range through a fast Fourier transform and generate a first detection result, determine whether a signal variance of the channel state information data set is greater than a preset variation amplitude and generate a second detection result, and determine whether the number of packets in the channel state information data set is less than a preset packet threshold and generate a third detection result, and to determine that a person is present when one of the first detection result, the second detection result, and the third detection result is true.

2. The personnel presence detection device as claimed in claim 1, wherein the signal receiving module is configured to receive the plurality of Wi-Fi reflected electromagnetic wave signals through a plurality of radio-frequency paths, and the computing module is configured to cluster the plurality of Wi-Fi reflected electromagnetic wave signals based on an intensity distribution of each Wi-Fi reflected electromagnetic wave signal in a plurality of subcarriers, and use the radio-frequency path with the largest number of packets as the main radio-frequency path.

3. The personnel presence detection device as claimed in claim 1, wherein the computing module is configured to cluster the plurality of Wi-Fi reflected electromagnetic wave signals based on a DBSCAN algorithm, and use the radio-frequency path with the largest number of packets as the main radio-frequency path.

4. The personnel presence detection device as claimed in claim 1, wherein the respiratory frequency range is from 8 to 22bpm.

5. The personnel presence detection device as claimed in claim 1, wherein the computing module is implemented in a cloud and connected to the processor through a wired network or a wireless network.

6. The personnel presence detection device as claimed in claim 1, further comprising:
a transmission module connected to the computing module, and the transmission module configured to transmit the detection results with personnel presence outward.

7. A personnel presence detection system, comprising:

   a first Wi-Fi device configured to continuously transmit a plurality of Wi-Fi electromagnetic wave signals,
   a second Wi-Fi device configured to continuously receive a plurality of Wi-Fi reflected electromagnetic wave signals, and obtain channel state information data based on the plurality of Wi-Fi reflected electromagnetic wave signals, and
   a computing module connected to the second Wi-Fi device, and the computing module configured to receive the channel state information data and identify a main radio-frequency path from the channel state information data, wherein the main radio-frequency path comprises a channel state information data set with the largest number of packets in the channel state information data,
   wherein the computing module is configured to determine whether the highest frequency value of the channel state information data set falls within a respiratory frequency range through a fast Fourier transform and generate a first detection result, determine whether a signal variance of the channel state information data set is greater than a preset variation amplitude and generate a second detection result, and determine whether the number of packets in the channel state information data set is less than a preset packet threshold and generate a third detection result, and to determine that a person is present when one of the first detection result, the second detection result, and the third detection result is true.

8. The personnel presence detection system as claimed in claim 7, wherein the second Wi-Fi device is configured to receive the plurality of Wi-Fi reflected electromagnetic wave signals through a plurality of radio-frequency paths, and the computing module is configured to cluster the plurality of Wi-Fi reflected electromagnetic wave signals based on an intensity distribution of each Wi-Fi reflected electromagnetic wave signal in a plurality of subcarriers, and use the radio-frequency path with the largest number of packets as the main radio-frequency path.

9. The personnel presence detection system as claimed in claim 7, wherein the computing module is configured to cluster the plurality of Wi-Fi reflected electromagnetic wave signals based on a DBSCAN algorithm, and use the radio-frequency path with the largest number of packets as the main radio-frequency path.

10. The personnel presence detection system as claimed in claim 7, further comprising a remote device; wherein the computing module is implemented in a cloud and connected to the second Wi-Fi device through a wired network or a wireless network, and the remote device is configured to connect to the computing module through the wired network and the wireless network to query the detection results with personnel presence.

11. A personnel presence detection method, comprising steps of:

   (a) continuously transmitting a plurality of Wi-Fi electromagnetic wave signals outward,
   (b) continuously receiving, by a Wi-Fi device, a plurality of Wi-Fi reflected electromagnetic wave signals,
   (c) obtaining, by the Wi-Fi device, channel state information data based on the plurality of Wi-Fi reflected electromagnetic wave signals,
   (d) identifying, by a computing module, a main radio-frequency path from the channel state information data, wherein the main radio-frequency path comprises a channel state information data set with the largest number of packets in the channel state information data,

(e) determining whether the highest frequency value of the channel state information data set falls within a respiratory frequency range through a fast Fourier transform and generate a first detection result,

(f) determining whether a signal variance of the channel state information data set is greater than a preset variation amplitude and generate a second detection result,

(g) determining whether the number of packets in the channel state information data set is less than a preset packet threshold and generate a third detection result, and

(h) determining that a person is present when one of the first detection result, the second detection result, and the third detection result is true.

12. The personnel presence detection method as claimed in claim 11, wherein step (a) is that the Wi-Fi device is configured to continuously transmit the plurality of Wi-Fi electromagnetic wave signals outward, and step (b) is that the same Wi-Fi device is configured to continuously receive the plurality of Wi-Fi reflected electromagnetic wave signals.

13. The personnel presence detection method as claimed in claim 11, wherein step (b) comprises a step of: receiving the plurality of Wi-Fi reflected electromagnetic wave signals through a plurality of radio-frequency paths, and step (d) comprises a step of: clustering the plurality of Wi-Fi reflected electromagnetic wave signals based on an intensity distribution of each Wi-Fi reflected electromagnetic wave signal in a plurality of subcarriers, and using the radio-frequency path with the largest number of packets as the main radio-frequency path.

14. The personnel presence detection method as claimed in claim 11, wherein step (d) comprises a step of: clustering the plurality of Wi-Fi reflected electromagnetic wave signals based on a DBSCAN algorithm, and using the radio-frequency path with the largest number of packets as the main radio-frequency path.

15. The personnel presence detection method as claimed in claim 11, further comprising a step of:

(i) connecting the computing module to a remote device through a wired network or a wireless network, and querying the detection results with personnel presence.

FIG. 1

FIG. 2

FIG. 3

start

continuously transmitting a plurality of Wi-Fi
electromagnetic wave signals — S40

continuously receiving a plurality of Wi-Fi
reflected electromagnetic wave signals — S41

obtaining channel state information (CSI) data
based on the plurality of Wi-Fi reflected
electromagnetic wave signals — S42

identifying a main radio-frequency path from
the CSI data — S43

determining whether the highest frequency value in the CSI data
set falls within a respiratory frequency range by using a fast
Fourier transform to generate a first detection result — S44

determining whether the signal variance of the CSI data set is
greater than a preset variation amplitude threshold, and generating
a corresponding second detection result — S45

determining whether the number of the signal packets included
in the CSI data set is less than a preset packet number
threshold, and generating a corresponding third detection result — S46

S49

no ← determining whether one of the detection results is true — S47

determining that the person is
not present in the confined space

yes

determining that the person is present
in the confined space — S48

receiving a connection and query from a remote
device to provide a detection result — S50

end

FIG. 4

FIG. 5

EP 4 746 314 A1

FIG. 6

FIG. 7B

FIG. 7A

FIG. 7D

FIG. 7C

20

FIG. 8

FIG. 9

EP 4 746 314 A1

FIG. 10

**EP 4 746 314 A1**

## INTERNATIONAL SEARCH REPORT

| International application No. |
|---|
| **PCT/CN2023/110868** |

### A. CLASSIFICATION OF SUBJECT MATTER

H04B10/071(2013.01)i; A61B5/11(2006.01)i

According to International Patent Classification (IPC) or to both national classification and IPC

### B. FIELDS SEARCHED

Minimum documentation searched (classification system followed by classification symbols)

IPC:H04B,A61B

Documentation searched other than minimum documentation to the extent that such documents are included in the fields searched

Electronic data base consulted during the international search (name of data base and, where practicable, search terms used)

CNTXT, CNABS, DWPI, VEN, USTXT, WOTXT, EPTXT, CJFD, 3GPP, CNKI, IEEE: 人员, 人体, 生命, 生物, 监测, 检测, 通讯状态信息, 通信状态信息, 信道状态信息, 运动, 动作, 活动, 呼吸, human, body, person, monitor, detect, channel state information, CSI, wifi, wi-fi, motion, movement, breath

### C. DOCUMENTS CONSIDERED TO BE RELEVANT

| Category* | Citation of document, with indication, where appropriate, of the relevant passages | Relevant to claim No. |
|---|---|---|
| A | CN 114285449 A (CHINA ELECTRONIC TECHNOLOGY NANHU INSTITUTE OF RESEARCH) 05 April 2022 (2022-04-05) description, paragraphs [0037]-[0044], and figure 1 | 1-15 |
| A | CN 114079859 A (CHINA UNIVERSITY OF MINING AND TECHNOLOGY) 22 February 2022 (2022-02-22) entire document | 1-15 |
| A | WO 2022096292 A1 (MERCEDES-BENZ GROUP AG) 12 May 2022 (2022-05-12) entire document | 1-15 |
| A | JP 2019148428 A (SAMSUNG ELECTRONICS CO., LTD.) 05 September 2019 (2019-09-05) entire document | 1-15 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **28 September 2023** | **04 January 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/110868**

| Patent document cited in search report | | | Publication date (day/month/year) | Patent family member(s) | | | Publication date (day/month/year) |
|---|---|---|---|---|---|---|---|
| CN | 114285449 | A | 05 April 2022 | None | | | |
| CN | 114079859 | A | 22 February 2022 | None | | | |
| WO | 2022096292 | A1 | 12 May 2022 | DE | 102020006878 | A1 | 12 May 2022 |
| JP | 2019148428 | A | 05 September 2019 | None | | | |

Form PCT/ISA/210 (patent family annex) (July 2022)